# EUROPEAN PATENT APPLICATION

(11) **EP 4 796 528 A1**
(43) Date of publication of application: **26.08.2026**
(21) Application number: 25159602.9
(22) Date of filing: 24.02.2025
(51) Int. Cl.: C07C 235/20, C07C 235/22, C07C 259/06, C07D 207/08, C07F 9/53, C22B 26/12

(54) **A MOLECULAR RECEPTOR, A METHOD FOR PREPARATION OF THE MOLECULAR RECEPTOR, A METHOD FOR THE SELECTIVE EXTRACTION OF LITHIUM AND A USE OF THE MOLECULAR RECEPTOR**

(71) Applicant: Instytut Chemii Organicznej Polskiej Akademii Nauk, 01-224 Warszawa (PL)
(72) Inventor: Kajetan, Dabrowa, 01-229 Warszawa (PL); Jakub, Narodowiec, 00-388 Warszawa (PL); Aleksandra, Kazimierczak, 01-143 Warszawa (PL); Malgorzata, Grela, 01-471 Warszawa (PL)
(74) Representative: AOMB Polska Sp. z.o.o.

(57) **Abstract**

The present invention relates to the field of lithium extraction and recycling. More specifically, the invention focuses on enhancing the selective recovery of monovalent lithium salts using molecular organic receptors with oxygen-atom donors.

The invention relates to a molecular receptor of general Formula 1, in which n, A, R₁-R₄, and B are defined in the description, a method for the preparation of the molecular receptor, a method for the selective extraction of lithium from a solid phase into an organic phase using the molecular receptor, and a use of the molecular receptor for the recovery of lithium from primary and secondary sources, including the recycling of lithium-ion batteries.

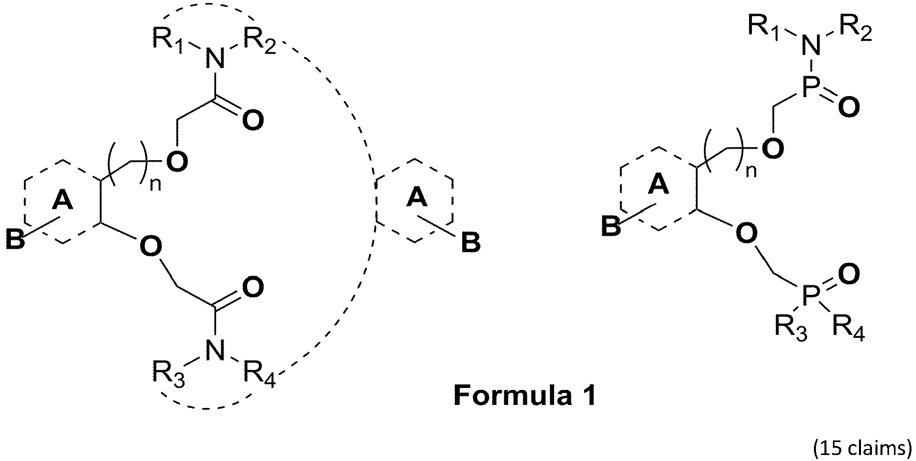

## Description

### Technical Field

The present invention relates to the field of lithium extraction and recycling. More specifically, the invention focuses on enhancing the selective recovery of monovalent lithium salts using molecular organic receptors with oxygen-atom donors. These systems enable efficient lithium recovery from brines and spent lithium-ion batteries, even in the presence of interfering cations.

### Prior Art

Over the past two decades, global lithium demand has significantly increased due to its essential role in modern materials, pharmaceuticals, and lithium-ion batteries (LIBs) ((a) Nature 2008, 451, 652-657, doi: 10.1038/451652a. (b) J. Am. Chem. Soc. 2013, 135(4), 1167-1176, doi: 10.1021/ja3091438). However, lithium reserves are finite, and some estimates suggest that readily accessible resources may not meet future demand *(*Procedic CIRP 2021, 98, 559-564, doi: 10.1016/j.procir.2021.01.151). Additionally, with lithium recycling rates remaining below 5% *(*Sep. Purif. Technol. 2017, 172, 388-403, doi: 10.1016/j.seppur.2016.08.031), new recovery strategies are needed, particularly from alternative sources such as brines and spent lithium-ion batteries.

Lithium extraction methods, including solid-liquid extraction (SLE) and liquid-liquid extraction (LLE), present promising solutions but face challenges due to the high lattice and hydration energies of lithium salts (-834 kJ-mol⁻¹ and -475 kJ·mol⁻¹ for Li⁺, -340 kJ·mol⁻¹ for Cl⁻ ions, respectively) ((a) Lide, David R., ed. CRC Handbook of Chemistry and Physics. Vol. 85. CRC press, 2004**.** (b) J. Chem. Soc., Faraday Trans. 1991, 87, 2995-2999, doi: 10.1039/FT9918702995), as well as the presence of competing cations (Na⁺, K⁺, Mg²⁺), which are found in much higher concentrations in brines (a) J. Mater. Sci. 2021, 56, 16-63, doi: 10.1007/s10853-020-05019-1. *(b)* Energies 2021, 14, 6805, doi: 10.3390/en14206805). Traditional lithium ionophores, developed since the 1980s recognition (J. Am. Chem. Soc. 1985, 107, 3657-3668, doi: 10.1021/ja00298a041. (b) J. Am. Chem. Soc. 1984, 106, 2160-2171, doi: 10.1021/ja00319a042), primarily rely on lipophilic anions, which complicate the extraction process, while existing separation techniques - such as ion exchange, precipitation, and membrane-based separation - often suffer from inefficiencies, high energy consumption, and environmental concerns.

To address these challenges, research efforts have focused on developing novel lithium-selective molecular receptors for direct lithium extraction (DLE). Conventional systems, such as cryptands and crown ethers, exhibit limitations in lithium selectivity and release kinetics, and many require lipophilic anions for effective lithium recognition, limiting their industrial applicability ((a) Desalination 2003, 158, 221-224, doi: 10.1016/S0011-9164(03)00455-7. b) Processes 2018, 6, 55, doi: 10.3390/pr6050055). Supramolecular chemistry offers a promising approach, with ion-pair receptors capable of simultaneously binding lithium cations and their counterions, enhancing lithium extraction efficiency ((a) Chem. Sci. 2024, 15, 13958-13965, doi: 10.1039/D4SC03760J. (b) J. Am. Chem. Soc. 2016, 138, 31, 9779-9782, doi: 10.1021/jacs.6b05713).

Recent patent applications describe various approaches for selective lithium extraction. For instance, document WO2019226863A1 discloses calixpyrrole-based cryptands capable of extracting LiCl from both solid and liquid phases, demonstrating high selectivity over competing alkali and alkaline earth metal salts. However, despite their promising performance, the reported synthetic route is highly demanding, requiring five to six steps and ultimately achieving a low overall yield of only 1.2-2.5% from commercially available starting materials. This significant limitation raises concerns regarding the scalability and practicality of the approach.

Document WO2023015138A1 presents a method utilizing lithium-selective sorbents based on strapped calixpyrroles, enabling lithium salt recovery via methanol elution and providing an effective means of separating lithium from other ions. However, the synthesis of the key monomers required for these sorbents is highly inefficient, involving six steps with overall yields of only 0.3-0.8% for H1-monomer and 0.4-1% for H2-monomer. This low synthetic efficiency significantly limits the practical applicability of the approach.

Similarly, the study by Heo et al. (Chem. Sci. 2024, 15, 13958-13965, doi: 10.1039/D4SC03760J) reports an ion pair receptor based on a phenanthroline-strapped calix[4]pyrrole framework, which selectively extracts LiCl and LiBr under solid-to-liquid (SLE) and liquid-to-liquid (LLE) extraction conditions. While the receptor demonstrates promising lithium salt binding properties, its synthesis requires four steps with an overall yield of just 0.9%, posing a substantial limitation for large-scale applications.

Furthermore, recent scientific advancements in SLE-based lithium extraction highlight the growing interest in optimizing lithium recovery processes. While calixpyrrole-based extractants remain a focus of research, few new lithium-selective hosts have emerged beyond cryptand-based receptors. Although these systems offer high selectivity, their complex synthesis and limited scalability pose significant challenges.

There is a clear need for simpler receptor architectures that can be produced cost-effectively on a large scale. Moreover, effective lithium extractants should be soluble in nonpolar solvents, a property often difficult to achieve with existing designs. These limitations highlight the ongoing need for advanced lithium recovery technologies to meet growing industrial demand.

### Summary of the Invention

According to the invention the first subject of the invention is a molecular receptor of the general Formula 1: 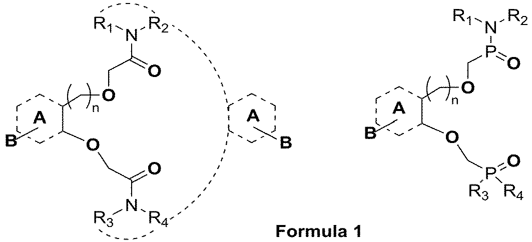 wherein:
n is an integer from 0 to 1;
A is an aromatic ring selected from the group comprising:
   phenyl, naphthyl, biphenyl, terphenyl, anthracenyl , phenanthrenyl, pyrenyl, benzothiophenyl, benzofuranyl, indolyl, quinolinyl, isoquinolinyl, benzimidazolyl, benzotriazolyl, benzoxazolyl, benzothiazolyl; pyrrolyl, furanyl, thiophenyl, oxazolyl, thiazolyl, imidazolyl, pyrazolyl, isoxazolyl, isothiazolyl, triazolyl, tetrazolyl, pyridyl, pyrazinyl, pyrimidinyl, pyridazinyl, triazinyl, azepinyl, oxepinyl, thiepinyl, purinyl, pteridinyl, carbazolyl, acridinyl, phenoxazinyl, phenothiazinyl;
B is a hydrogen or at least one substituent independently classified as:
   (a) Electron-withdrawing groups (EWG), defined as functional groups with a Hammett substituent constant (*σₚₐᵣₐ*) greater than or equal to +0.10 (σ*ₚₐᵣₐ* ≥ +0.10), including but not limited to -NO₂, -CN, -SCF₃, -SOCF₃, -CF₃, -SO₂R⁵, -COR⁵, -COOR⁵ (where R⁵ is independently selected from hydrogen (H), alkanediyl (C≤18), aryl, or heteroaryl), amide (-CONR⁶R⁷, where R⁶ and R⁷ are independently selected from H, alkanediyl (C≤18), aryl, or heteroaryl), urea (-NR⁸CONR⁹R¹⁰, where R⁸, R⁹, and R¹⁰ are independently H, alkanediyl (C≤18), aryl, or heteroaryl), thioamide (-CSNR¹¹R¹², where R¹¹ and R¹² are independently selected from H, alkanediyl (C≤18), aryl, or heteroaryl), thiourea (-NR¹³CSNR¹⁴R¹⁵, where R¹³, R¹⁴, and R¹⁵ are independently selected from H, alkanediyl (C≤18), aryl, or heteroaryl), and halogens;
   (b) Electron-donating groups (EDG), defined as functional groups with a Hammett substituent constant (*σₚₐᵣₐ*) less than -0.10 (*σₚₐᵣₐ <* -0.10), including but not limited to -OR¹⁶, -SR¹⁶, -NR¹⁶R¹⁷, and alkyl groups (C≤18), wherein R¹⁶ and R¹⁷ are independently selected from hydrogen (H), alkanediyl (C≤18), aryl, or heteroaryl, positioned at the ortho, meta, or para positions relative to aromatic ring structure,
R₁-R₄ are independently alkanediyl (C≤24), cycloalkanediyl (C≤12), arenediyl (C≤24), substituted arenediyl (C≤24), heteroarenediyl (C≤24), substituted heteroarenediyl (C≤8) or at least two of R₁-R₄ are taken together to form a group of the formula C: 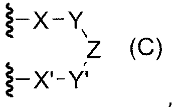 wherein:
   X and X' are each independently alkanediyl (C≤8), cycloalkanediyl (C≤8), alkenediyl (C≤8), alkynediyl (C≤8), arenediyl (C≤8), or a substituted version of any of these groups;
   Y and Y' are each independently -O-, -C(=O)-, -C(=O)O-, -S(=O)-, -S(=O)₂O-, -C(=O)NR¹⁸-, or - S(=O)₂NR¹⁹-, wherein: R¹⁸ and R¹⁹ are each independently hydrogen, alkanediyl (C≤6), or substituted alkanediyl (C≤6);
   Z is an alkanediyl (C≤8), alkanediyl (C≤8), cycloalkanediyl (C≤8), alkenediyl (C≤8), alkynediyl (C≤8), arenediyl (C≤8), or a substituted version of any of these groups;

in proviso that when n = 0, at least one of R₁-R₄ is other than cyclohexane.

Preferably the compound of Formula 1 is selected from:

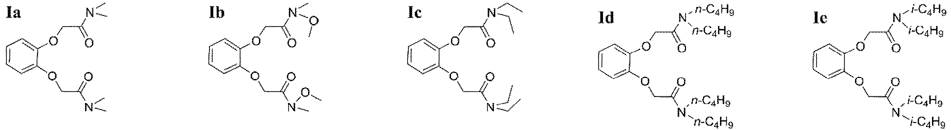

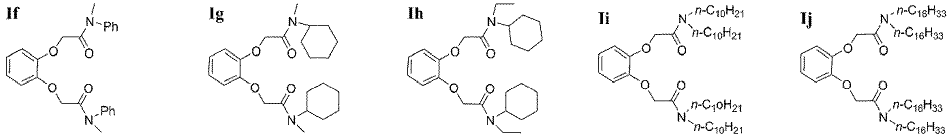

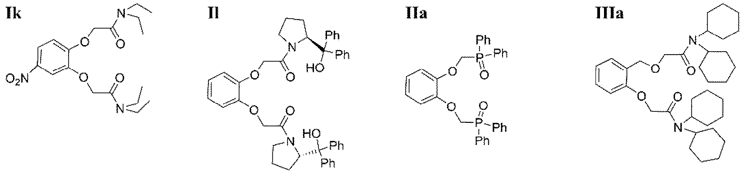

Another aspect of the invention is a method for preparation of the molecular receptor of Formula 1, comprising the step of:

### Reacting a compound of formula

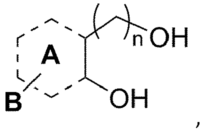

wherein
n is an integer ranging from 0 to 1;
A and B are defined above,
with the corresponding alkylating agent of general formula Y-CH₂C(=O)NR₁R₂ or Y-CH₂P(=O)R₁R₂, where Y = Cl, Br, or I and R₁, R₂ are defined above,
wherein the reaction is carried out for 1 to 96 hours in aprotic solvent or in a heterogeneous
mixture at 0-210 °C in the presence of base.

Preferably the method is optionally conducted under phase transfer catalysis (PTC) conditions.

Preferably the aprotic solvent is selected from the group comprising: acetonitrile, dichloromethane, dichloroethane, dimethylformamide, dimethylacetamide, benzene, toluene, *ortho*-xylene, *meta-*xylene, *para*-xylene, tetrahydrofuran, 2-methyltetrahydrofuran, 1,2-dimethoxyethane, 1,4-dioxane, *tert*-butyl methyl ether, N-methylpyrrolidone, dimethylsulfoxide, or the mixture thereof.

Preferably the heterogeneous mixture comprises: dichloromethane, dichloroethane, benzene, toluene, *ortho*-xylene, *meta*-xylene, *para*-xylene, *tert*-butyl methyl ether, 2-methyltetrahydrofuran, or the mixture thereof as the organic phase, and the aqueous solution of alkali metal hydroxides. Preferably the base is selected from the group comprising alkali metal carbonates (M₂CO₃), alkali metal hydroxides (MOH), alkali metal hydrides (MH), and alkali metal phosphates (M₃PO₄), where M = Li, Na, K, Cs, and alkali metal alkoxides (ROM), where M = Na, K, and R is a C₁-C₄ alkyl group, specifically selected from methyl, ethyl, n-propyl and isopropyl.

Preferably the alkali metal carbonates (M₂CO₃), alkali metal hydroxides (MOH) and alkali metal phosphates (M₃PO₄) are in the solid form or as aqueous solution.

Yet another aspect of the invention is a method for the selective extraction of lithium from a solid phase into a liquid organic phase using the molecular receptor of Formula 1, comprising the steps of:
(a) Contacting the solid material containing lithium salts with a solution of the molecular receptor in the range of 0.001-2 mol/L in a liquid organic phase;
(b) Allowing the system to equilibrate from 1 second to 30 days to enable lithium transfer from the solid phase into the liquid organic phase;
(c) Separating the liquid organic phase from the solid residue by decantation, filtration, or centrifugation;
(d) Optionally filtering the organic phase to remove any suspended particulates,
wherein the whole process is conducted at a temperature ranging from 0°C to 100°C, preferably between 20°C and 40°C.

Preferably the solid material is selected from individual lithium salts, salt mixtures, or spent lithium-ion battery materials.

Preferably the extraction process is conducted under controlled mixing conditions, including continuous agitation, intermittent shaking, or passive equilibration over time.

Preferably the process is performed in a batch-wise or continuous mode.

Preferably the liquid organic phase is selected from the group comprising chlorinated hydrocarbons, branched chlorinated hydrocarbons (C₄-C₁₈), hydrocarbons (C₅-C₁₇), branched hydrocarbons (C₅-C₁₈), toluene, benzene, nitrobenzene, *ortho*-xylene, *meta*-xylene, *para*-xylene, or the mixture thereof. Preferably a solid phase is selected from the group comprising lithium salts.

Yet another aspect of the invention is a use of the molecular receptor of Formula 1 for the recovery of lithium from primary and secondary sources, including the recycling of lithium-ion batteries.

The present invention introduces structurally simple, molecular receptors that enable the selective solubilization of lithium salts from solid materials with low lithium content (1200 ppm) into non-water-miscible aprotic solvents. Unlike conventional macrocyclic or cryptand-type systems, which are often complex, costly, and difficult to scale up, the disclosed systems are synthesized in a few straightforward steps from inexpensive, commercially available precursors. This eliminates the need for costly reagents and labor-intensive synthetic procedures, making the approach highly cost-effective and industrially viable. While a structurally related compound (Sodium lonophore III, ETH 2120) was previously known as a sodium-selective receptor, subsequent studies revealed its strong affinity for lithium salts. However, the novel derivatives described in this invention exhibit significantly improved extraction efficiency and selectivity. Their ability to solubilize lithium salts efficiently has been demonstrated using spent lithium-ion batteries and a synthetic brine mixture formulated to replicate the natural salt composition of the Salar de Atacama, where lithium must be selectively extracted from a high-salinity environment dominated by sodium and magnesium salts. Furthermore, the molecular framework of these molecular systems allows for functional modifications to enhance their solubility in nonpolar solvents, further optimizing their performance in industrial applications. Unlike conventional lithium-selective ionophores, which often require additional counter-anionic components or exhibit slow lithium release kinetics, the present invention offers a more efficient and scalable solution. These features make the invention particularly valuable for large-scale lithium recovery, providing an economically and environmentally sustainable alternative to traditional extraction methods.

### Brief Description of the Drawings

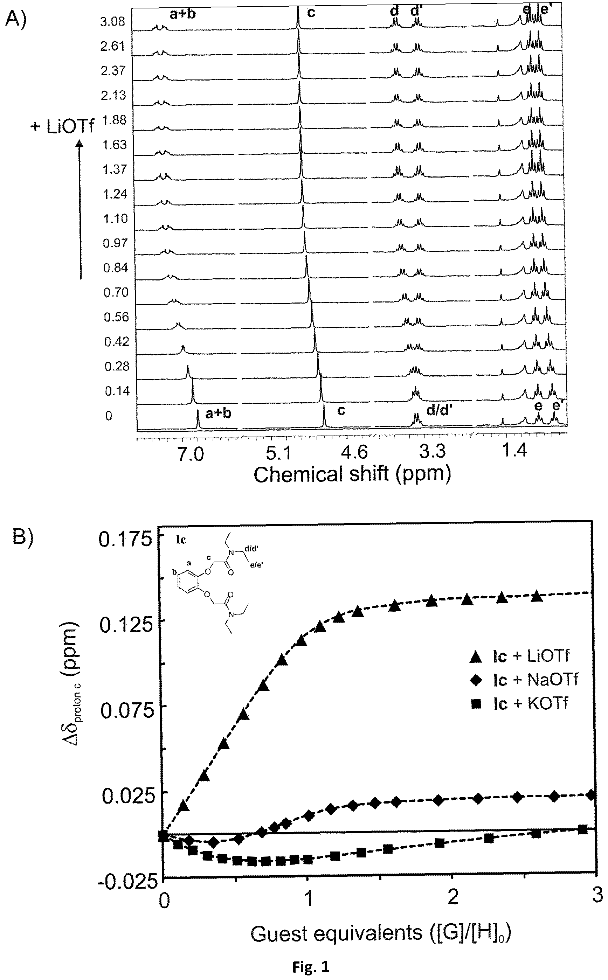

The following figures are incorporated as part of this specification and are included to further demonstrate certain aspects of the present disclosure. The disclosure can be better understood by reference to one or more of these drawings in conjunction with the detailed description of the specific examples of implementation provided herein:
Figs. 1A-B show partial ¹H NMR titration spectra of receptor **Ic** in CDCl₃, illustrating chemical shift changes upon incremental addition of LiOTf (Fig. 1A). The corresponding binding isotherms for **Ic** with LiOTf, NaOTf, and KOTf are presented in Fig. 1B, showing the chemical shift change (Δδ) of proton c as a function of guest equivalents. The molecular structure of **Ic** with labeled protons is depicted in the inset.
Figs. 2A, C show partial ¹H and ⁷Li NMR spectra from SLE experiments after mixing a 10.0 mM **Ic** solution in CDCl₃ with excess salt, salt mixture, or spent Li-ion battery material. Fig. 2B depicts the molecular structure of **Ic** with proton labeling.
Figs. 3A, C show partial ¹H and ⁷Li NMR spectra from SLE experiments after mixing a 10.0 mM **Ie** solution in CDCl₃ with excess salt, salt mixture, or spent Li-ion battery material. Fig. 3B depicts the molecular structure of **Ie** with proton labeling.
Figs. 4A, C show partial ¹H and ⁷Li NMR spectra from SLE experiments after mixing a 10.0 mM **Ii** solution in CDCl₃ with excess salt, salt mixture, or spent Li-ion battery material. The molecular structure of the receptor **Ii** and proton labeling are depicted in FIG. 2B.
Figs. 5A, C show partial ¹H and ⁷Li NMR spectra from SLE experiments after mixing a 10.0 mM **IIIa** solution in CD₂Cl₂ with excess salt, salt mixture, or spent Li-ion battery material. The molecular structure of the receptor **IIIa** and proton labeling are depicted in FIG. 5B.
Fig. 6 presents the front (FIGs. 1A, C, E) and side (FIGs. 1B, D, F) views of the DFT-calculated structures of **Ic** complexed with lithium salts in different solvents using the C-PCM model.
Specifically, FIGs. 1A-B depict the **Ic**/CF₃SO₃Li complex in acetonitrile, FIGs. 1C-D show **Ic**/LiCl·H₂O in acetonitrile, and FIGs. 1E-F illustrate **Ic**/LiCl·H₂O in chloroform. Selected hydrogen bond lengths (dashed lines) are provided in the inset tables. Lithium and chloride ions are represented in the space-fill model. For clarity, receptor protons are omitted.

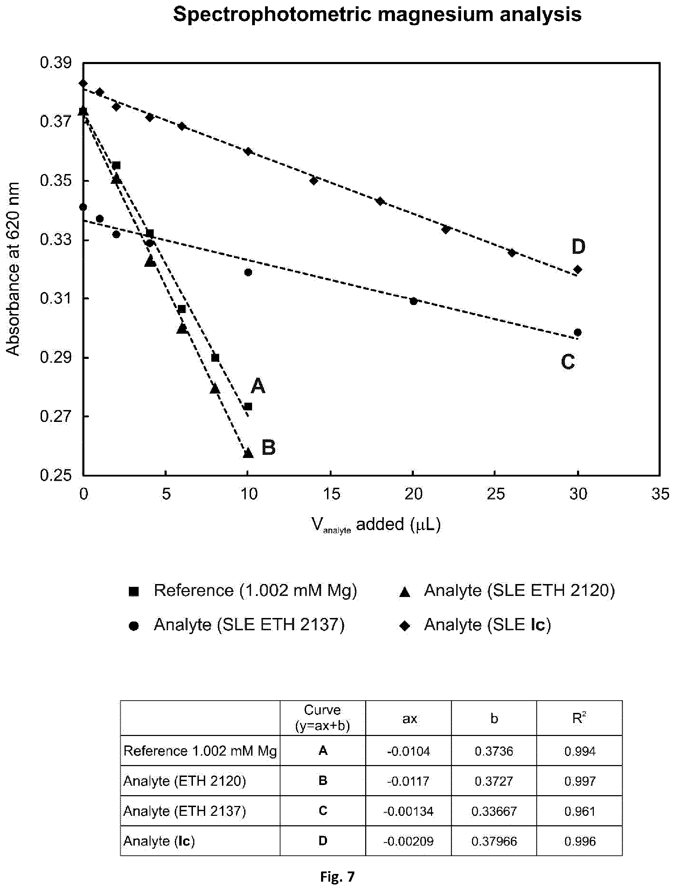

Fig. 7 presents the spectrophotometric quantification of Magnesium co-extraction in Solid-Liquid Extraction (SLE) Experiments with Linear Fit Parameters and R² Values.

### Examples

### 1. Preparation of molecular receptors

The molecular receptors I-IV disclosed in this patent can be obtained by simple chemical transformations, and three general synthetic methods are described (Scheme 1). Depending on the chosen method, the corresponding receptor can be synthesized at a scale exceeding 50 mmol, with moderate to high yields. Method (a) involves O-alkylation of catechol derivatives with suitable bromo/chloro alkylating reagents (e.g., X-CH₂CONR₁R₂ (**3**) or X-CH₂P(=O)R₁R₂ (**6**)) in the presence of K₂CO₃ in DMF. Method (b) uses the O-alkylation of salicyl alcohol derivatives with appropriate bromo/chloro alkylating reactants in the presence of NaH in DMF. Method (c) utilizes the reaction of secondary amines with catechol-derived acid dichloride prepared in a three-step procedure described in the literature. The molecular receptors I-III synthesized using these methods were fully characterized using NMR spectroscopy (¹H, ¹³C, ³¹P) and high-resolution mass spectrometry (HR-MS), and the analytical data are summarized in Table 1.

All reagents and solvents were used as received unless stated otherwise. Reaction progress was monitored by TLC on silica gel 60 F254 plates (Merck). Products were purified by column chromatography on Kieselgel 60 (Merck), with flash chromatography used when necessary. Compound identity and purity were confirmed by ¹H NMR, ¹³C, ³¹P NMR, and MS (ESI-MS/APCI-MS) spectroscopy. NMR spectra were recorded on Bruker 400 (Avance III HD) and Varian-Agilent 600 MHz (vnmrs) instruments, with chemical shifts referenced to TMS (δ 0.00 ppm), CDCl₃ (¹H: δ 7.26 ppm, ¹³C: δ 77.16 ppm), or CD₂Cl₂ (¹H: δ 5.32 ppm, ¹³C: δ 54.00 ppm). Data include chemical shifts, multiplicity (s, d, dd, t, m), coupling constants (Hz), and integration. ESI/APCI-MS spectra were recorded on an AutoSpec Premier spectrometer, and UV-vis spectra on a Varian Cary 60 spectrophotometer.

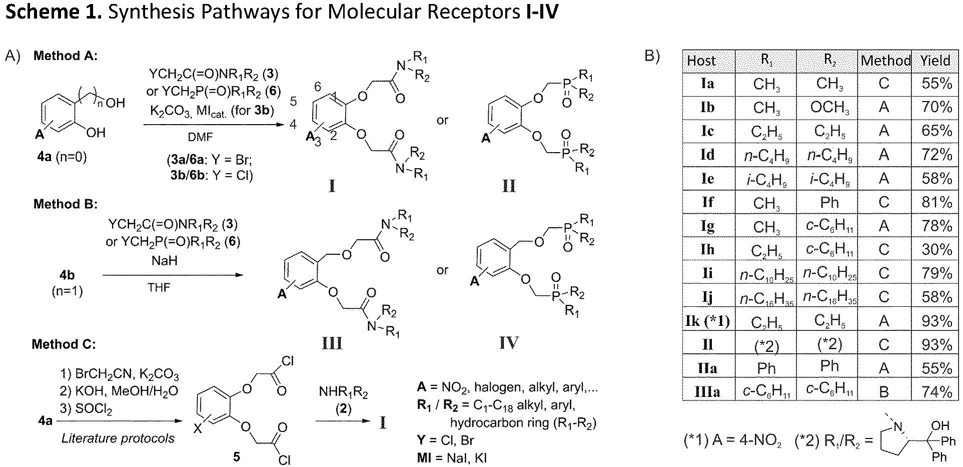

### 1.1. Method for the Synthesis of 2-Bromoacetamides (3a) Intermediates

To a solution of the corresponding amine **2** (1.0 equiv.) in dichloromethane (1.5 mL per mmol of amine), a solution of K₂CO₃ (1.5 equiv.) in distilled water (2 mL per mmol of amine) was added. The resulting biphasic mixture was cooled to 0°C, and 2-bromoacetyl bromide (1.5 equiv.) was added dropwise under vigorous stirring. After complete addition, the reaction mixture was removed from the cooling bath and stirred for 1 hour, allowing the reaction mixture to reach room temperature. The reaction mixture was then transferred to a separatory funnel, and the organic layer was separated. The aqueous layer was extracted with dichloromethane (0.5 mL per mmol of amine), and the combined organic phases were dried over Na₂SO₄ filtered, and concentrated under reduced pressure. The crude residue was purified by short-path silica gel chromatography using heptane/ethyl acetate mixture as eluent. In some cases, the product was further purified by recrystallization from hot heptane. Note: 2-Chloro-N,N-disubstituted acetamides (3b) are commercially available and can serve as alternative starting materials.

### 1.2. Method A for the Synthesis of Molecular Receptors (I and II)

A catechol derivative **4a** (1.0 equiv.), dry K₂CO₃ (3.0 equiv.), and NaI (0.1 equiv., only when **3b** or **6b** was used) were placed under argon in a round-bottom flask. Anhydrous DMF (4 mL per mmol of catechol derivative) was added, and the mixture was stirred for 5 minutes. Next, 2-halogen-N,N-disubstituted acetamide (**3a** or **3b,** 2.5 equiv.) or (halogenomethyl)-disubstitutedphosphine oxide (**6a** or **6b,** 2.5 equiv.) was added in one portion, and the reaction mixture was heated to 50°C (150°C in case of **6b)** and stirred overnight. After completion, the reaction mixture was concentrated under reduced pressure and the oily residue was dissolved in dichloromethane (4 mL per mmol of catechol derivative) and water (4 mL per mmol), and the resulting emulsion was transferred to a separatory funnel. The organic layer was separated, while the aqueous layer was extracted with dichloromethane (2 mL per mmol). The combined organic layers were dried over Na₂SO₄, filtered, and concentrated under reduced pressure. The crude product was purified by flash chromatography, followed by recrystallization from hot ethyl acetate or, when feasible, by slow evaporation from a dichloromethane/heptane mixture.

### 1.3. Method B for the Synthesis of Molecular Receptors (III and IV)

The corresponding salicylic alcohol derivative **4b** (1.0 equiv.) was placed in a round-bottom flask, and THF (6 mL per mmol of alcohol) was added. Sodium hydride (60% suspension in mineral oil, 2.5 equiv.) was then added portionwise under stirring. The reaction mixture was heated to 50°C for 1 hour, then cooled to 0°C, and the desired 2-bromo-N,N-disubstituted acetamide **3a** (3.0 equiv.) was added. The reaction mixture was then heated to 50°C and stirred for 4 days. Upon completion, the reaction was cooled to room temperature, quenched with saturated NH₄Cl_{(aq)}, and extracted with dichloromethane. The organic layer was dried over Na₂SO₄, filtered, and concentrated under reduced pressure. The resulting oily residue was purified by flash chromatography, affording the final product.

### 1.4. Method C for the Synthesis of Molecular Receptors (I)

The corresponding acid chloride intermediates **(5)** were prepared by adapting previously reported literature methods: for 1,2-phenylenedioxydiacetyl dichloride, see: (a) Tetrahedron 2005, 61, 5351-5362, doi: 10.1016/j.tet.2005.03.064. *(b)* Synthesis 2006, 6, 999-1004, doi: 10.1055/s-2006-926357; for 4-nitro-1,2-phenylenedioxydiacetyl dichloride, see: (c) J. Heterocyclic Chem. 1981, 18, 297, doi: 10.1002/jhet.5570180214o. *(d)* Dalton Trans. 2022, 51, 9039-9048, doi: 10.1039/D2DT01074G. The aryl diacetyl dichloride **5** (1.0 equiv.) was placed in a round-bottom flask under an argon atmosphere, dissolved in dichloromethane (5 mL per mmol of acid chloride), and cooled to 0°C. Triethylamine (5.78 equiv.) was then added, followed by the addition of the desired secondary amine **2** (3.0 equiv.). The reaction mixture was allowed to slowly warm to room temperature and stirred overnight. Upon completion, the reaction was transferred to a separatory funnel, and an aqueous solution of 2M HCl was added along with an additional portion of dichloromethane. The organic phase was collected, while the aqueous phase was extracted with dichloromethane (as needed). The combined organic layers were dried over Na₂SO₄, filtered, and concentrated under reduced pressure. The resulting oily residue was purified by flash chromatography, yielding the final product.

**Table 1. NMR Spectroscopic Data (¹H and ¹³C) and HRMS data for the molecular receptors (MR) I-III.**

| MR | NMR chemical shifts δ (ppm) and HRMS (ESI) data |
|---|---|
| **Ia** | ¹H NMR (400MHz, CDCl₃): δ (ppm) 6.99-6.90 (m, 4H), 4.75 (s, 4H), 3.10 (s, 6H), 2.97 (s, 6H). ¹³C NMR (100 MHz, CDCl₃): δ (ppm) 168.27, 148.54, 122.82, 115.67, 69.02, 36.92, 36.07. HRMS (ESI) calculated for C₁₄H₂₀N₂O₄Na [M+Na]⁺: 303.1321, found: 303.1324. |
| **Ib** | ¹H NMR (400 MHz, CDCl₃): δ (ppm) 6.97-6.89 (m, 4H), 4.90 (s, 4H), 3.73 (s, 6H), 3.20 (s, 6H). ¹³C NMR (100 MHz, CDCl₃): 169.94, 148.92, 122.73, 116.40, 67.56, 61.95, 32.65. HRMS (ESI) calculated for C₁₈H₂₈N₂O₄Na [M+Na]⁺: 359.1951, found: 359.1947 |
| **Ic** | ¹H NMR (400 MHz, CDCl₃): 6.86-6.96 (m, 4H), 4.70 (s, 4H), 3.32-3.41 (m, 8H), 1.17 (t, *J* = 7.1 Hz, 6H), 1.10 (t, *J* = 7.1 Hz, 6H). ¹³C NMR (100 MHz, CDCl₃): 167.04, 148.32, 122.29, 115.19, 68.78, 41.50, 40.23, 14.30, 12.81. HRMS (ESI) calculated for C₁₄H₂₀N₂O₆Na [M+Na]⁺: 335.1219, found: 335.1221 |
| **Id** | ¹H NMR (400 MHz, CDCl₃): 6.97-6.86 (m, 4H), 4.73 (s, 4H), 3.30 (q, *J* = 7.0 Hz, 8H), 1.60 - 1.44 (m, 8H), 1.36-1.21 (m, 8H), 0.91 (t, *J* = 7.3 Hz, 6H), 0.89 (t, *J* = 7.3 Hz, 6H). ¹³C NMR (100 MHz, CDCl₃): 167.46, 148.45, 122.32, 115.39, 68.61, 47.07, 45.73, 31.15, 29.66, 20.27, 20.17, 13.94, 13.91. |
| | HRMS (ESI) calculated for C₂₆H₄₄N₂O₄Na [M+Na]⁺: 471.3199, found: 471.3202. |
| **Ie** | ¹H NMR (400 MHz, CDCl₃): 6.98 - 6.85 (m, 4H), 4.77 (s, 4H), 3.19 (d, *J* = 7.7 Hz, 4H), 3.17 (d, *J* = 7.8 Hz, 5H), 2.04-1.86 (m, 4H), 0.90 (d, *J* = 6.7 Hz, 12H), 0.80 (d, *J* = 6.7 Hz, 12H). ¹³C NMR (100 MHz, CDCl₃): 168.37, 148.46, 122.33, 115.62, 68.69, 54.37, 52.66, 27.43, 26.23, 20.16, 20.09. |
| | HRMS (ESI) calculated for C₂₆H₄₄N₂O₄Na [M+Na]⁺: 471.3199, found: 471.3202. |
| **If** | ¹H NMR (400 MHz, CDCl₃): 7.43-7.19 (m, 10H), 6.86-6.69 (m, 4H), 4.41 (s, 4H), 3.28 (s, 6H). ¹³C NMR (100 MHz, CDCl₃): 167.74, 148.67, 142.34, 129.94, 128.24, 127.13, 122.30, 116.27, 67.98, 37.43. HRMS (ESI) calculated for C₂₄H₂₄N₂O₆Na [M+Na]⁺: 427.1634, found: 427.1637. |
| **Ig** | ¹H NMR (400 MHz, CDCl₃): 6.97-6.84 (m, 4H), 4.76-4.66 (m, 4H), 4.42-4.26 (m, 1H), 3.70-3.58 (m, 1H), 2.87 (s, 3H), 2.79 (s, 3H), 1.85-0.96 (m, 20H). ¹³C NMR (101 MHz, CDCl₃) *δ* (ppm) δ 167.55, 167.42, 167.37, 167.29, 148.28, 148.24, 148.23, 148.10, 122.31, 122.23, 122.20, 122.10, 115.50, 115.12, 115.10, 114.57, 69.12, 69.02, 68.90, 68.70, 56.45, 56.36, 52.91, 52.86, 31.84, 30.96, 29.57, 28.98, 28.85, 27.40, 25.63, 25.55, 25.51, 25.25, 22.66 (Note: three conformers stable within NMR timescale are present). HRMS (ESI) calculated for C₂₄H₃₆N₂O₄Na [M+Na]⁺: 439.2573, found: 439.2578. |
| **Ih** | ¹H NMR (600 MHz, CDCl₃): 7.01-6.88 (m, 4H), 4.77-4.70 (m, 4H), 4.24-4.15 (m, 1H), 3.72-3.63 (m, 1H), 3.36-3.26 (m, 4H), 1.84-1.02 (m, 26H). ¹³C NMR (151 MHz, CDCl₃): 167.52, 167.36, 167.25, 167.06, 148.71, 148.61, 148.42, 148.18, 122.42, 122.30, 122.27, 122.09, 115.81, 115.37, 115.26, 114.48, 69.36, 69.10, 68.94, 68.72, 57.16, 56.99, 54.84, 54.76, 38.02, 37.98, 36.93, 32.05, 32.02, 30.81, 26.05, 25.93, 25.71, 25.43, 25.41, 16.87, 14.83, 14.78 (Note: three conformers stable within NMR timescale are present). HRMS (ESI) calculated for C₂₆H₄₀N₂O₄Na [M+Na]⁺: 467.2886, found: 467.2873. |
| **Ii** | ¹H NMR (600 MHz, CDCl₃): 7.01-6.88 (m, 4H), 4.77-4.70 (m, 4H), 4.24-4.15 (m, 1H), 3.72-3.63 (m, 1H), 3.36-3.26 (m, 4H), 1.84-1.02 (m, 26H). ¹³C NMR (151 MHz, CDCl₃): 167.52, 167.36, 167.25, 167.06, 148.71, 148.61, 148.42, 148.18, 122.42, 122.30, 122.27, 122.09, 115.81, 115.37, 115.26, 114.48, 69.36, 69.10, 68.94, 68.72, 57.16, 56.99, 54.84, 54.76, 38.02, 37.98, 36.93, 32.05, 32.02, 30.81, 26.05, 25.93, 25.71, 25.43, 25.41, 16.87, 14.83, 14.78. HRMS (ESI) calculated for C₅₀H₉₂N₂O₄Na [M+Na]⁺: 807.6955, found: 807.6948. |
| **Ij** | ¹H NMR (600 MHz, CDCl₃): 6.96 - 6.88 (m, 4H), 4.74 (s, 4H), 3.30 (q, *J* = 8.0 Hz, 8H), 1.60 - 1.47 (m, 9H), 1.33-1.19 (m, 104H), 0.88 (t, *J* = 7.0 Hz, 12H). ¹³C NMR (151 MHz, CDCl₃): 167.49, 148.52, 122.35, 115.46, 68.67, 47.31, 46.05, 32.08, 29.86, 29.85, 29.83, 29.82, 29.77, 29.58, 29.57, 29.52, 29.15, 27.65, 27.18, 27.08, 22.84, 14.26. HRMS (ESI) calculated for C₇₄H₁₄₁N₂O₄ [M+H]⁺: 1122.0891, found: 1122.0889. |
| **Ik** | ¹H NMR (400 MHz, CDCl₃): 7.83 (dd, *J* = 8.9, 2.6 Hz, 1H), 7.71 (d, *J* = 2.6 Hz, 1H), 6.95 (d, *J* = 9.0 Hz, 1H), 4.85 (s, 2H), 4.81 (s, 2H), 3.28-3.42 (m, 8H), 1.03-1.26 (m, 12H). ¹³C NMR (100 MHz, CDCl₃): 165.74, 165.64, 153.61, 147.81, 141.87, 118.33, 113.13, 109.53, 68.04, 67.55, 41.43, 41.36, 40.43, 40.37, 14.31, 14.26, 12.84, 12.78. HRMS (ESI) calculated for C₁₈H₂₇N₃O₆Na [M+Na]⁺: 404.1798, found: 404.1800. |
| **II** | ¹H NMR (600 MHz, CDCl₃)*:* 7.41-7.19 (m, 20H), 6.98-6.93 (m, 2H), 6.80 (dd, *J* = 5.9, 3.7 Hz, 2H), 6.47 (s, 2H), 5.24-5.18 (m, 2H), 4.67 (dd, *J* = 104.1, 14.7 Hz, 4H), 3.50 (q, *J* = 8.8 Hz, 2H), 3.03 (q, *J* = 7.8 Hz, 2H), 2.09-2.01 (m, 2H), 2.00-1.92 (m, 2H), 1.64-1.54 (m, 2H), 1.33-1.22 (m, 2H), 1.10-1.01 (m, 2H). ¹³C NMR (151 MHz, CDCl₃): 170.59, 148.19, 146.21, 143.24, 128.11, 128.09, 127.79, 127.66, 127.45, 127.43, 122.97, 115.81, 81.77, 69.09, 67.88, 47.61, 32.02, 29.31, 29.16, 23.66, 22.83, 14.25. HRMS (ESI) calculated for C₄₄H₄₄N₂O₆Na [M+Na]⁺: 719.3097, found: 719.3100. |
| **IIa** | ¹H NMR (400 MHz, CDCl₃): 7.86-7.74 (m, 8H), 7.54-7.33 (m, 12H), 6.93-6.80 (m, 4H), 4.62 (d, *J* = 7.0 Hz, 4H). ¹³C NMR (100 MHz, CDCl₃): 149.02 (d, *J* = 11.0 Hz), 132.42 (d, *J* = 2.8 Hz), 131.58 (d, *J* = 9.5 Hz), 130.72 (d, *J* = 101.1 Hz), 128.77 (d, *J* = 11.9 Hz), 122.94, 115.06, 67.61 (d, *J* = 87.2 Hz). |
| | ³¹P NMR (162 MHz, CDCl₃): 26.21. HRMS (ESI) calculated for C₃₂H₂₈O₄NaP₂ [M+Na]⁺: 561.1361, found: 561.1357. |
| **IIIa** | ¹H NMR (600 MHz, CDCl₃): 7.42 (dd, J = 7.6, 1.8 Hz, 1H), 7.22 (td, *J* = 7.8, 1.8 Hz, 1H), 6.96 (t, *J* = 7.4 Hz, 1H), 6.90 (d, *J* = 8.2 Hz, 1H), 4.68 (s, 2H), 4.64 (s, 2H), 4.15 (s, 2H), 3.57 (t, *J* = 11.2 Hz, 1H), 3.50 (t, *J* = 11.5 Hz, 1H), 2.93 (bs, 2H), 2.60-2.37 (m, 4H), 1.86-1.02 (m, 40H). ¹³C NMR (151 MHz, CDCl₃): 168.35, 167.15, 155.79, 128.98, 128.78, 126.45, 121.24, 111.56, 71.62, 69.58, 67.73, 57.81, 57.30, 56.47, 56.19, 34.25, 32.01, 31.50, 31.44, 30.02, 29.86, 29.14, 26.76, 26.68, 26.02, 25.97, 25.48, 25.41, 25.33, 22.82, 22.46, 14.24, 14.18. HRMS (ESI) calculated for C₃₅H₅₄N₂O₄Na [M+Na]⁺: 589.3981, found: 589.3983. |

### 2. Determination of Complexation Properties: Binding Constants and Computational Analysis

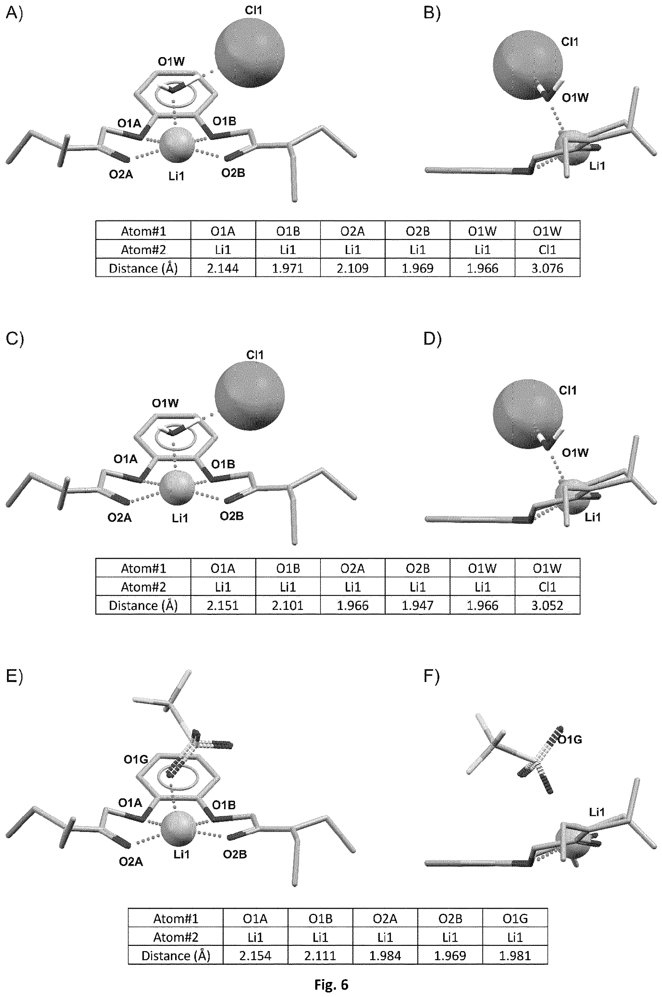

NMR and UV-Vis titrations were conducted in liquid solution to determine the relative cation selectivity of the obtained systems for Li⁺, Na⁺, K⁺, and Mg²⁺. For comparison, also Sodium lonophore III (ETH 2120) and Benzo-12-crown-4 ether (B12C4) were included. To minimize the influence of the counterion, triflate salts were used due to their good solubility in the employed solvent system (MeCN + H₂O, 99.5/0.5% v/v). The results of these studies are summarized in Table 2, with example titration data for Ic shown in Fig. 1. These measurements clearly indicate that, among alkali metal cations, all obtained systems exhibit selectivity toward lithium, following the trend Li⁺ > Na⁺ > K⁺. A similar selectivity pattern was observed for the ETH2120 reference compound, whereas B12C4 displayed a different selectivity sequence of Na⁺ > K⁺ > Li⁺. When compared to the divalent Mg²⁺ cation, all receptors exhibited significantly stronger interactions with Mg²⁺, with the selectivity trend being Mg²⁺ >> Li⁺ > Na⁺ > K⁺. Furthermore, it was observed that for Na⁺, K⁺, and Mg²⁺ cations, the described systems tend to form 2:1 (host:guest) complexes. In contrast, the obtained molecular extractants for lithium generally do not exhibit this tendency, except for compound **IIa.** The observed binding selectivity is supported by theoretical calculations, which indicate that lithium salts form more stable complexes compared to sodium and potassium. These computations were performed using density functional theory (DFT) at a high level of theory, specifically employing the M06-2X functional with the def2-TZVP basis set and solvent approximations (MeCN and CHCl₃). The calculation details are provided in the description, and the results are included in Fig. 6 and are summarized in Table 4. The DFT-calculated structure for the complex of receptor **Ic** with LiCl·H₂O confirms strong lithium binding within the receptor's cleft. The lithium cation is coordinated by four oxygen donors from the receptor and one water molecule, while the chloride counterion interacts only with water, aligning with the water peak shift observed in SLE experiments. Calculations indicate a similar binding mode for Ic in its complexes with LiOTf (FIGs. 6E-F) and LiCl·H₂O (FIGs. 6A-D). Moreover, the **Ic**/LiCl·H₂O structure in C-PCM chloroform (matching the solvent used in extraction experiments) closely resembles those in acetonitrile, as evidenced by comparable hydrogen bond lengths listed in Fig. 6. This indicates a high degree of preorganization of the host structure.

### 2.1. NMR Titration Procedure

Solution of the corresponding molecular receptor (Ia, Ic-Ie, Ih, Ii, IIa, IIIa, Sodium lonophore III (ETH2120), and benzo-12-crown-4 ether (B12C4)) was prepared by dissolving precisely weighted (± 0.02 mg precision) sample of receptor in appropriate volume of 99.5% MeCN-*d*₃ + 0.5% H₂O (v/v) mixture (± 0.002 mL precision), yielding a ^{~}10 mM solution, used directly for titration with NaOTf and KOTf. For titration with LiOTf, solution was diluted 10 times with a 99.5% MeCN-*d*₃ + 0.5% H₂O (v/v) mixture providing a final concentration ~1mM. Aliquote of the given triflate was dissolved in the solution of the receptor. 0.5 mL of the receptor solution was placed in a screw-cap NMR tube, and solution of the given triflate was added stepwise *via* microliter syringe, with ¹H NMR spectra recorded at 400 MHz after every addition (~20-25 additions per titration). Collected titration data were fitted with Musketeer software *(*Chem. Sci. 2024, 15, 15299-15310. doi: 10.1039/D4SC03354J), providing binding constants given in Table 2.

### 2.2. UV-Vis Titration Procedure

Solution of the corresponding molecular receptor (Ic-Ie, Ih, Ii, IIIa, and Sodium lonophore III (ETH2120)) was prepared by dissolving precisely weighted (± 0.02 mg precision) sample of the receptor in appropriate volume of 99.5% MeCN + 0.5% H₂O (v/v) mixture (± 0.002 mL precision), yielding a ^{~}10 mM stock solution. Aliquote of the given metal triflate salt was dissolved in 99.5% MeCN + 0.5% H₂O (v/v) mixture. In a screw-cap quartz cuvette with 1 cm path length, precisely a 2.475 mL of 99.5% MeCN + 0.5% H₂O (v/v) mixture was placed, background UV-Vis spectrum was recorded, and 25 µL of the stock solution of the receptor was added. Spectrum of the pure receptor was recorded, and solution of Mg(OTf)₂ was added stepwise *via* microliter syringe, with UV-Vis spectra recorded after every addition (~20-25 additions per titration). Collected data were fitted with BindFit v0.5 software (https://supramolecular.org, Chem. Commun. 2016, 52, 12792-12805, doi: 10.1039/C6CC03888C), providing binding constants given in Table 2.

**Table 2. Stability constants (logKₐ) for Molecular Receptors (la, Ic, Id, Ie, Ih, Ii, Ila, and Illa), along with Sodium lonophore III (ETH 2120) and Benzo-12-crown-4 ether (B12C4) for comparison**

| Receptor | LiOTf^{[a], [b]} | NaOTf^{[a], [c]} | KOTf^{[a],[c]} | Mg(OTf)₂^{[f]} |
|---|---|---|---|---|
| **Ia** | 4.23 | ^{~}3.4^{[d]} | 2.29 (3.62)^{[e]} | - ^{[g]} |
| **Ic** | 4.60 | ^{~}3.6^{[d]} | 2.3 (2.29)^{[e]} | 6.2 (10.8) |
| **Id** | 4.66 | ^{~}3.5^{[d]} | 2.36 (3.73)^{[e]} | 6.3 (12.5) |
| **Ie** | 4.3 | ^{~}3.4^{[d]} | 2.35 (3.33)^{[e]} | 5.5 (10.9) |
| **Ih** | 4.76 | ^{~}3.4^{[d]} | 2.43 (3.99) | 6.2 (12.5) |
| **Ii** | 4.98 | 3.72 (6.19) | 2.36 (4.47) | 4.3 (10.5) |
| **IIa** | 3.28 (5.23)^{[c]} | 2.0 (3.69)^{[e]} | 1.31 | - ^{[h]} |
| **IIIa** | 4.72 | 2.93 (4.65) | 1.63 | 5.5 (10.6) |
| **ETH2120** | 4.33 | 3.68 (5.72) | 2.39 (4.09) | 6.8 (13.7) |
| **B12C4** | 1.44^{[c]} | 1.97 (4.19)^{[e]} | 1.78 | <1^{[a], [c]} |

| | | | | |
|---|---|---|---|---|
| [a] Determined using ¹H titrations in 99.5% CD₃CN + 0.5% (v/v) H₂O solvent mixture at 298 K; *K*_{a,1} is in M⁻¹, cations added as triflate (CF₃SO₃) salts; estimated *K* errors ±10%; in parentheses *K*_{2:1} host:guest ([M⁻²]). [b] Measured at ~1 mM receptor concentration. [c] Measured at ^{~}10 mM receptor concentration. [d] Problem with fitting to 2:1 model. [e] *K*_{2:1} uncertain. [f] Determined using UV-Vis titrations at ~0.1 mM receptor concentration in 99.5% CH₃CN + 0.5% (v/v) H₂O solvent mixture at 298 K; *K*_{a,1} is in M⁻¹, cations added as triflate (CF₃SO₃) salts; estimated K errors ±20%; in parentheses *K*_{2:1} host:guest ([M⁻²]). [g] Not determined. [h] Complex binding mode. | | | | |

### 2.3. Theoretical Calculations of Complexation Properties

Energy-minimized structures of free molecular receptors and their complexes with alkali metal (Li, Na, K) and magnesium triflates and chlorides were obtained by adopting a previous protocol (Chem. Commun. 2014, 50, 15748-15751, doi: 10.1039/C4CC07798A). The structures with lowest energies were subjected to optimization and frequency calculations using Gaussian 16 (Rev A. 03) program with M06- 2X *(*Theor. Chem. Acc. 2007, 120, 215-241, doi: 10.1007/s00214-007-0310-x) functional, which demonstrated to have a good performance for describing noncovalent interactions and thermochemistry. Structure optimization and frequency calculations were performed at the Def2-TZVP level of theory and C-PCM method *(*J. Chem. Theory Comput. 2005, 1, 70-77, doi: 10.1021/ct049977a) for the solvent calculations. Solvated free energies were calculated as Gsolv = ESCF + corrₜₕₑᵣₘ, where ESCF is taken from the output file of the solvent calculation (under: "SCF DONE: E(R_functional) = XX") and corr therm is taken from the output of the frequency calculation (under: "Thermal correction to Gibbs Free Energy = XX"). The binding energies (G_{bind}) were calculated as G_{complex} - (G_{receptor} + Gₛₐₗₜ), where G_{complex}, G_{receptor} and Gₛₐₗₜ are the solvated free energies for the complex, receptor and salt, respectively. All final optimized structures were verified by vibrational frequency calculations, confirming the absence of imaginary frequencies, which indicates that the obtained geometries correspond to true minima on the potential energy surface.

**Table 4. Calculated free Gibbs Binding Enthalpies (G_{bind}) for receptor Ic with guests in solvents^{[a]}**

| Solvent: acetonitrile (ε = 35.7) (G_{receptor} = -1112.7549^{[b]}) | | | | |
|---|---|---|---|---|
| Guest | LiOTf | NaOTf | KOTf | Mg(OTf)₂ |
| G_{compound}^{[b]} | -969.2114 | -1123.9558 | -1561.6085 | -2123.3324 |
| G_{complex}^{[b]} | -2081.9861 | -2236.7205 | -2674.3732 | -3236.1628 |
| G_{bind}^{[c]} | -12.5 | -6.2 | -6.2 | -47.5 |

| Guest | LiCl/H₂O | NaCl/H₂O | KCl/H₂O | MgCl₂ |
|---|---|---|---|---|
| G_{compound}^{[b]} | -544,3073 | -699,0484 | -1136,7010 | -1120.6946 |
| G_{complex}^{[b]} | -1657.0785 | -1811.8152 | -2249.4649 | -2233.4824 |
| G_{bind}^{[c]} | -10.3 | -7.5 | -5.7 | -20.7 |

| Solvent: chloroform (TCMε = 4.8) (G_{receptor} = -1112.7549^{[b]}) | | | | |
|---|---|---|---|---|
| Guest | LiCl/H₂O | NaCl/H₂O | KCl/H₂O | MgCl₂ |
| G_{compound}^{[b]} | -544.2956 | -699.0370 | -1136.6914 | -1120.6794 |
| G_{complex}^{[b]} | -1657.0677 | -1811.8048 | -2249.4543 | -2233.4824 |
| G_{bind}^{[c]} | -12.8 | -10.2 | -7.1 | -32.3 |

| | | | | |
|---|---|---|---|---|
| [a] Calculated at the DFT/M06-2X/def2-TZVP level of theory using Gaussian 16 (Rev A. 03), with the solvent environment approximated by the C-PCM model. [b] In Hartree (au). [c] In kcal/mol. | | | | |

### 3. Extraction of Lithium Salts from Primary and Secondary Sources

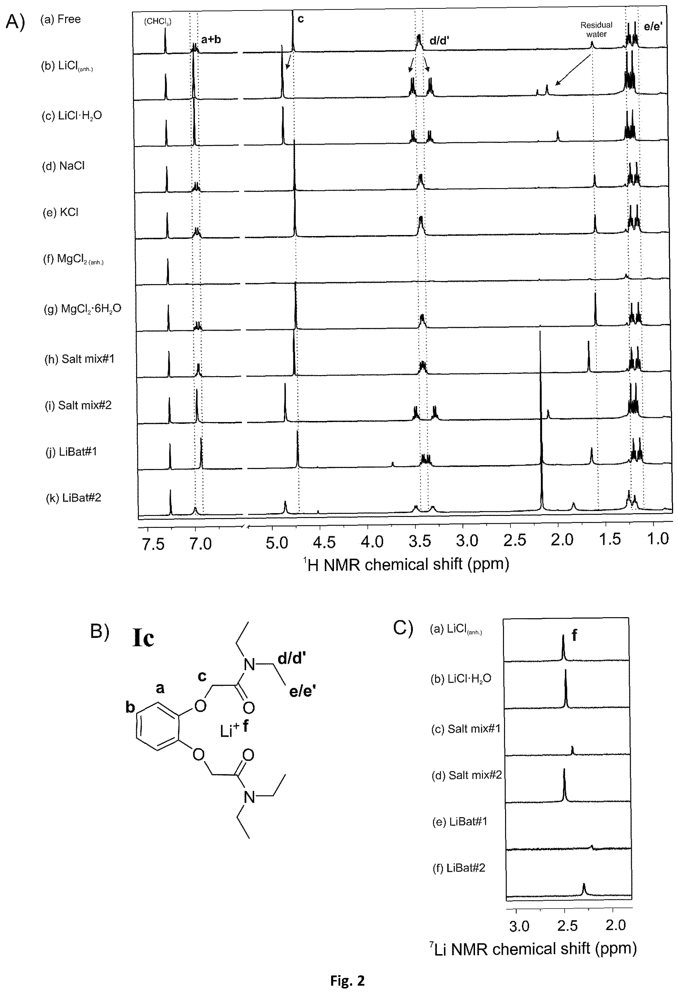

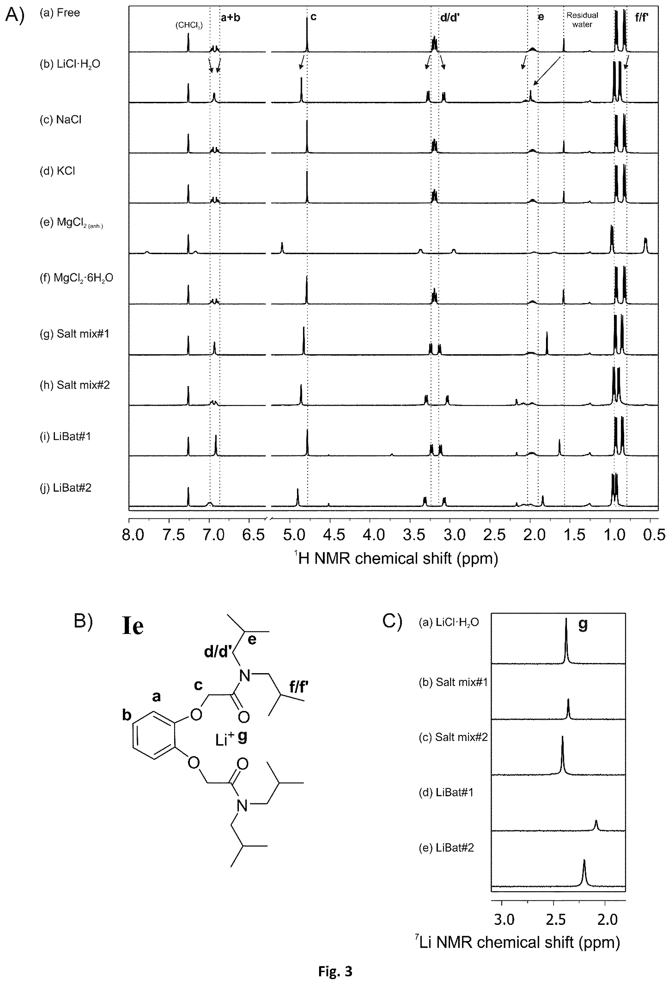

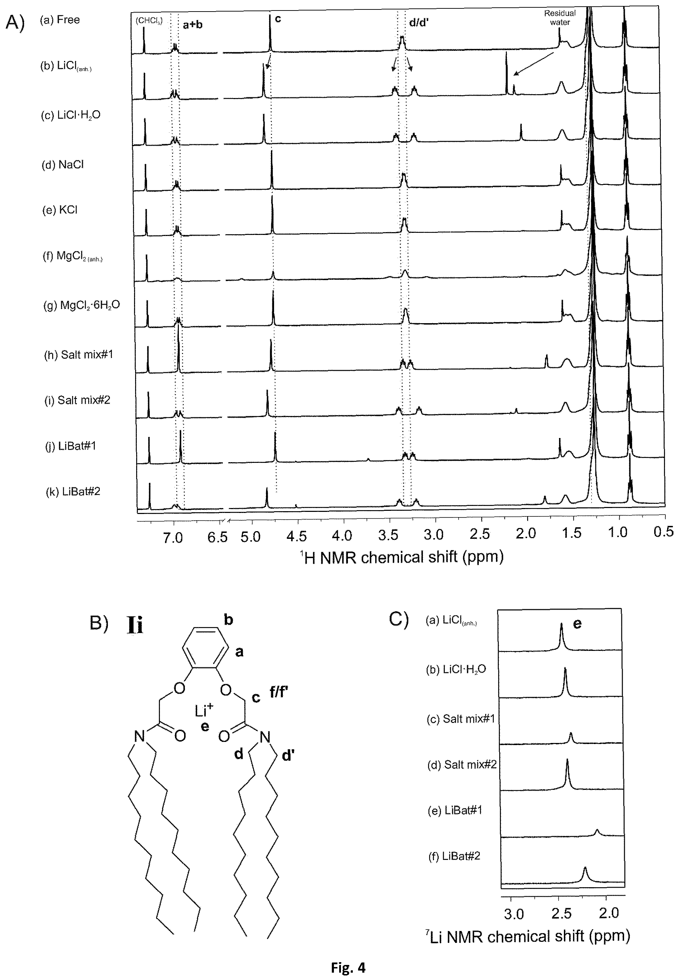

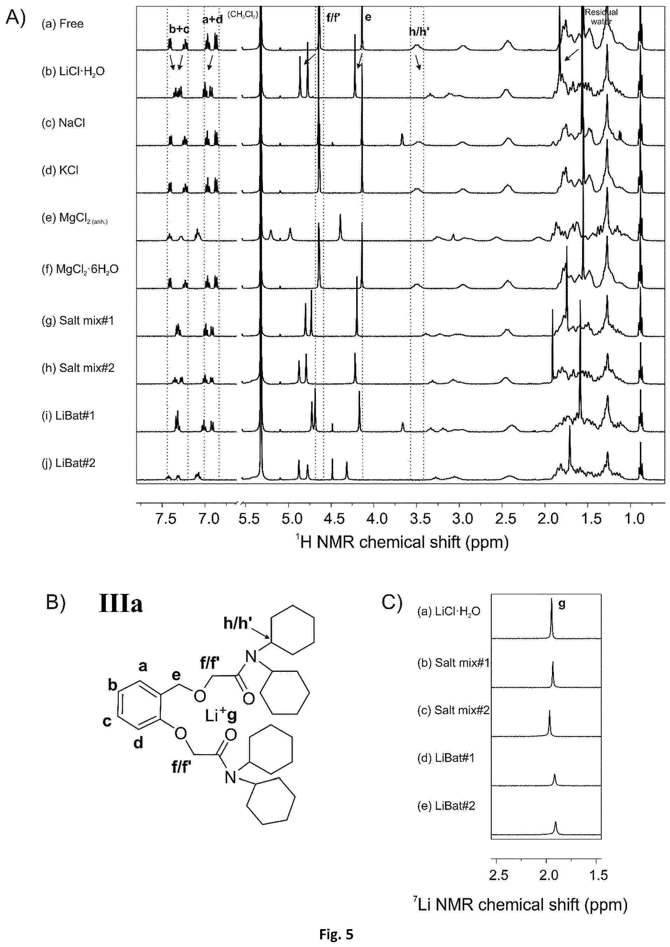

To investigate whether the obtained molecular receptors are capable of selectively extracting lithium salts from the solid to liquid phase, a series of solid-to-liquid (SLE) extraction experiments were performed. Representative results for molecular receptors **Ic, Ie, Ii,** and **IIIa** are depicted in Figs. 2-5. All tested receptors exhibited similar extraction properties, confirming the general applicability of the observed trends. The extracted material-a solution of the receptor in halogenated hydrocarbon solvents (CD₂Cl₂ and CDCl₃)-was analyzed using NMR spectroscopy (¹H and ⁷Li). These halogenated hydrocarbons were chosen due to their immiscibility with water and their preference in industrial applications. In these solvents, alkali metal chloride salts and MgCl₂ are practically insoluble. By comparing the spectra of the free receptor before and after extraction, it is possible to determine whether the receptor successfully extracted a given salt from the solid material. If extraction occurs, a receptor/salt complex is formed, significantly altering the proton environment of the receptor, as reflected in the shift of signals in the ion-pair binding region. Additionally, the measurement of ⁷Li NMR unambiguously confirms whether lithium salt is transferred into the solution (⁷Li NMR signals are generally broad due to the high quadrupole moment of the ⁷Li nucleus). No shift changes were observed in SLE experiments with NaCl, KCl, and MgCl₂·6H₂O salts (Figs. 2-5). However, significant changes were detected in the extraction of anhydrous LiCl, LiCl·H₂O, a mixture containing 1200 ppm lithium in NaCl/KCl (Salt mix#1), and a highly hydrated mixture of LiCl with MgCl₂, NaCl, and KCl (Salt mix#2, containing 20 wt% water, prepared to mimic the composition of brine from Salar de Atacama, one of the world's largest lithium deposits), as well as from two different lithium-ion batteries materials such as LCO/LMCO-type (Spent Li-Ion battery#1) and LFP-type (Spent Li-Ion battery#2). The details of solid samples used in SLE experiments are described in Table 3. Larger spectral shifts were observed for the LFP-type batteries. Furthermore, in the ⁷Li NMR spectra, only one sharp signal was detected, indicating that lithium is strongly bound and the complex is rigid. Additionally, the obtained extractants successfully extracted anhydrous MgCl₂ but not its hydrated form. Notably, from the MgCl₂-containing mixture, lithium was preferentially extracted, while magnesium extraction remained minimal (2.0%), comparable to the lithium benchmark, Lithium lonophore IV (ETH 2137, CAS 108083-23-4), which exhibited an extraction efficiency of 1.6%. This was determined by spectrophotometric studies using Eriochrome Black T (details in the patent description). These results unambiguously confirm the high selectivity of the molecular receptors described in this invention for the selective extraction of lithium salts, even from the hydrated sources containing high content of interfering cations.

**Table 3. Overview of Solid Samples, Manufacturer, and Composition**

| No | Material | Description & manufacturer | Purity (%wt) |
|---|---|---|---|
| 1 | LiCl (anh.) | Anhydrous, free-flowing, Sigma-Aldrich (MKCS7062, 1003596143) | 99% |
| 2 | LiCl·H₂O | Lithium chloride monohydrate, Angene (Lot: AGN23-1260018-1) | 99% |
| 3 | NaCl | Sodium chloride, pure for analysis, Stanlab (47/12/23) | 99.9% |
| 4 | KCl | Potassium chloride, EMSURE for analysis, Merck (K49198936 747) | 99.5% |
| 5 | MgCl₂ (anh.) | Anhydrous, Sigma (079K0080) | 98% |
| 6 | MgCl₂·6H₂O | Magnesium chloride, pure for analysis, Stanlab (36/04/24) | 99.9% |
| 7 | Salt Mix 1 | Physical mixture containing 1200 ppm lithium (equivalent to 0.74 wt.% LiCl) in Na/K chlorides, see rows 2-4 | LiCl: 0.74%, NaCl: 97.6%, KCl: 1.31%, H₂O: 0.31% |
| 8 | Salt Mix 2 | Physical wet mixture containing 4.4% wt of LiCl in NaCl/KCl/MgCl₂ (Salar de Atacama), see rows 2-4 and 6 | LiCl: 4.4%, NaCl: 44.1%, KCl: 15.6%, MgCl₂: 15.9%, H₂O: 20.0% |
| 9 | Spent Li-Ion battery#2 | Cathode material from various spent LiCoO₄ (LCO) and LiMnCO₄ (LMCO) batteries, various vendors. The Li content in this material is within the range of 5.5-6.5 wt.%, consistent with values reported in the literature for similar battery compositions. | |
| 10 | Spent Li-Ion battery#1 | Cathode material from spent LiFePO₄ (LFP) batteries, China vendor, IFR26650 LFP 3.2V 3.4Ah 10.88Wh. The Li content in this material is within the range of 3.5-5.5 wt.%, consistent with values reported in the literature for similar battery compositions. | |

### 3.1. Preparation of Salt Mix 1

LiCl·H₂O (101 mg), KCI (126 mg), and NaCl (9.802 g) were dissolved in ~25 mL of hot water. Solution was then concentrated on a rotary evaporator (~20 mbar, 100°C) to solid residue, which was transferred to mortar and ground.

### 3.2. Preparation of Salt Mix 2

LiCl·H₂O (1.828 g), KCI (4.53 g), NaCl (12.78 g), and MgCl₂·6H₂O (9.82 g) were dissolved in ~50mL of hot water. Solution was then concentrated on a rotary evaporator (~20 mbar, 100°C) to solid residue (mass 28.90 g), which was transferred to mortar and ground.

### 3.3. Procedure for Disassembly, Separation, and Preparation of Spent Li-Ion Battery Materials

Each type of battery was systematically disassembled into its constituent layers, including an aluminum layer (anode), a copper layer (cathode), and a polymeric separator. The majority of the active material was found adhered to the aluminum and copper foils, which were selected for further processing. The aluminum and copper foils were separated, individually comminuted, and placed into reaction flasks, where they were subjected to continuous stirring using a magnetic stirrer for 12-15 hours (overnight). A cylindrical stirring bar was employed as an abrasive element to facilitate the detachment of the active material from the foil surfaces. The resulting black powder was subsequently sieved using a series of mesh screens, with the smallest mesh size being 200 µm. The fine fraction obtained after sieving was further processed by dispersing it in chloroform (100 mL per ^{~}10 g of black mass) under continuous magnetic stirring overnight. The resulting suspension was then filtered using a Schott funnel and dried under vacuum to yield purified active material.

### 3.4. SLE Extraction Protocol for Primary and Secondary Lithium Sources

Vials were prepared containing a weighted amount of individual salts (TAB. 4, rows 1-6, 10 molar excess per corresponding ME), salt mixtures (TAB. 4, rows 7-8, 50mg each), and spent Li-Ion batteries (TAB. 4, rows 9-10, 300mg each). For the vials containing salts 1-6, 0.7 mL of a 10 mM solution of ME in deacidified CDCl₃ was added, while for the vial containing the Li-Ion battery material, 0.7-1.0 mL of a 10 mM solution of ME in deacidified CDCl₃ was added. The samples were mixed for 2 hours at 40 rpm. The vial contents were then drawn into a syringe and filtered through a 0.45 µm PTFE syringe filter into NMR tubes, and spectrum was recorded.

### 3.5. Protocol for determining the relative content of co-extracted magnesium

The content of magnesium co-extracted during the solubilization of lithium chloride from a Salt Mix 2 into chlorinated aprotic solvents was quantitatively determined using a spectrophotometric method adapted from the procedure described by Harvey et al. (Anal. Chem. 1953, 25, 498-500, doi: 10.1021/ac60075a031). The analytes were aqueous solutions obtained *via* back-extraction from a 10 mM stock solution of the given host in CHCl₃, following solid-liquid extraction (SLE) with a Salar de Atacama reference sample (Table 3, Salt Mix #2). For spectrophotometric measurements, a screw-cap quartz cuvette with a 1 cm path length was used. First, 2 mL of a 10 mM LiCl aqueous solution was placed into the cuvette, followed by the addition of 0.5 mL of buffer (pH = 10.1, prepared by dissolving 675 mg of NH₄Cl in water, adding 5.7 mL of 25% aqueous ammonia, and diluting with water to 100 mL). A background spectrum was recorded before adding 25 µL of a 0.1% methanolic solution of Eriochrome Black T (CAS: 1787-61-7). After recording the initial spectrum, the analyte solution was added stepwise in microliter portions, with spectra recorded after each addition. The absorbance at 620 nm was plotted as a function of the added analyte volume (corrected for dilution), and the slope of the resulting linear function was compared to that obtained for a 1.002 mM reference MgCl₂ solution. The analyte concentration was determined using the proportional relationship: (c_{analyte} = c_{reference} × slope_{analyte} / slope_{reference}). This method eliminates dependence on the absolute concentration of Eriochrome Black T, which is critical due to the limited purity of commercial samples and the instability of its solutions. The measured magnesium concentrations were 0.20 ± 0.01 mM for **Ic,** 1.1 ± 0.05 mM for **IIIa,** and 0.16 ± 0.01 mM for ETH 2137 (Fig. 7). Since the back-extraction was performed using a 1:1 volume ratio of deionized water to the CHCl₃ solution, the estimated magnesium co-extraction into CHCl₃ during SLE corresponds to approximately 2 mol% relative to Ic, 11 mol% relative to **IIIa,** and 1.6 mol% relative to ETH 2137. For Ic, this results in a Li:Mg ratio of 98:2.

This research was funded in whole or in part by the National Science Centre, Poland, grant number 2022/47/B/ST5/01787.

## Claims

1. A molecular receptor of general Formula 1: 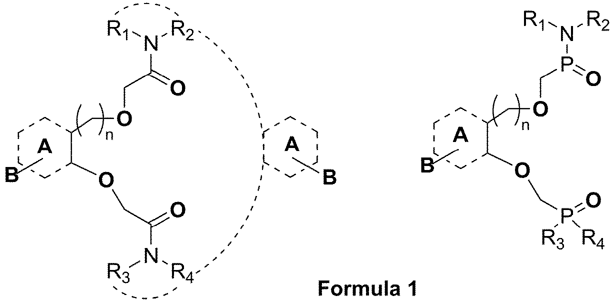 , wherein
n is an integer from 0 to 1;
A is an aromatic ring selected from the group comprising:
phenyl, naphthyl, biphenyl, terphenyl, anthracenyl, phenanthrenyl, pyrenyl, benzothiophenyl, benzofuranyl, indolyl, quinolinyl, isoquinolinyl, benzimidazolyl, benzotriazolyl, benzoxazolyl, benzothiazolyl; pyrrolyl, furanyl, thiophenyl, oxazolyl, thiazolyl, imidazolyl, pyrazolyl, isoxazolyl, isothiazolyl, triazolyl, tetrazolyl, pyridyl, pyrazinyl, pyrimidinyl, pyridazinyl, triazinyl, azepinyl, oxepinyl, thiepinyl, purinyl, pteridinyl, carbazolyl, acridinyl, phenoxazinyl, phenothiazinyl;
B is hydrogen or at least one substituent independently classified as:
(a) Electron-withdrawing groups (EWG), defined as functional groups with a Hammett substituent constant (*σₚₐᵣₐ*) greater than or equal to +0.10 (*σₚₐᵣₐ* ≥ +0.10), including but not limited to -NO₂, -CN, -SCF₃, -SOCF₃, -CF₃, -SO₂R⁵, -COR⁵, -COOR⁵ (where R⁵ is independently selected from hydrogen (H), alkanediyl (C≤18), aryl, or heteroaryl), amide (-CONR⁶R⁷, where R⁶ and R⁷ are independently selected from H, alkanediyl (C≤18), aryl, or heteroaryl), urea (-NR⁸CONR⁹R¹⁰, where R⁸, R⁹, and R¹⁰ are independently H, alkanediyl (C≤18), aryl, or heteroaryl), thioamide (-CSNR¹¹R¹², where R¹¹ and R¹² are independently selected from H, alkanediyl (C≤18), aryl, or heteroaryl), thiourea (-NR¹³CSNR¹⁴R¹⁵, where R¹³, R¹⁴, and R¹⁵ are independently selected from H, alkanediyl (C≤18), aryl, or heteroaryl), and halogens;
(b) Electron-donating groups (EDG), defined as functional groups with a Hammett substituent constant (*σₚₐᵣₐ*) less than -0.10 (*σₚₐᵣₐ* < -0.10), including but not limited to - OR¹⁶, -SR¹⁶, -NR¹⁶R¹⁷, and alkyl groups (C≤18), wherein R¹⁶ and R¹⁷ are independently selected from hydrogen (H), alkanediyl (C≤18), aryl, or heteroaryl, positioned at the ortho, meta, or para positions relative to aromatic ring structure,
R₁-R₄ are independently alkanediyl (C≤24), cycloalkanediyl (C≤12), arenediyl (C≤24), substituted arenediyl (C≤24), heteroarenediyl (C≤24), substituted heteroarenediyl (C≤8) or at least two of R₁-R₄ are taken together to form a group of the formula C: 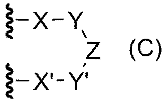 , wherein:
X and X' are each independently alkanediyl (C≤8), cycloalkanediyl (C≤8),
alkenediyl (C≤8), alkynediyl (C≤8), arenediyl (C≤8), or a substituted version of any of these groups;
Y and Y' are each independently -O-, -C(=O)-, -C(=O)O-, -S(=O)-, -S(=O)₂O-, - C(=O)NR¹⁸-, or -S(=O)₂NR¹⁹-, wherein: R¹⁸ and R¹⁹ are each independently hydrogen, alkanediyl (C≤6), or substituted alkanediyl (C≤6);
Z is an alkanediyl (C≤8), alkanediyl (C≤8), cycloalkanediyl (C≤8), alkenediyl (C≤8), alkynediyl (C≤8), arenediyl (C≤8), or a substituted version of any of these groups;
in proviso that when n = 0, at least one of R₁-R₄ is other than cyclohexane.

2. The molecular receptor according to claim 1, wherein the compound of Formula 1 is selected from:

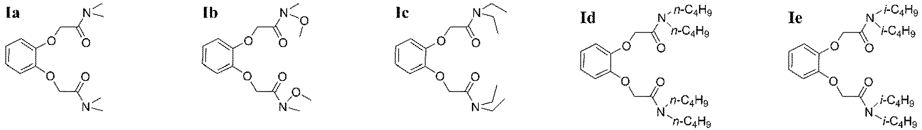

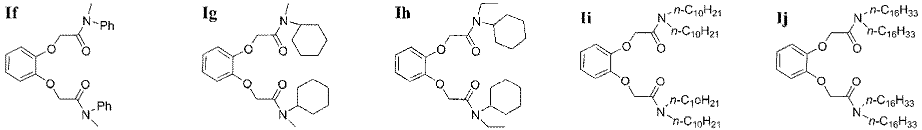

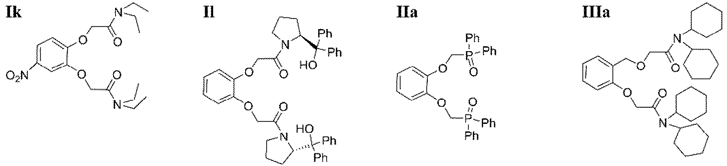

3. A method for preparation of the molecular receptor specified in claim 1 or 2, comprising the step of:
reacting a compound of formula 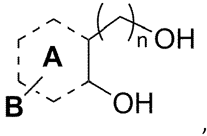 wherein
n is an integer ranging from 0 to 1;
A and B are defined above;
with the corresponding alkylating agent of general formula Y-CH₂C(=O)NR₁R₂ or Y-CH₂P(=O)R₁R₂, where Y = Cl, Br, or I and R₁, R₂ are defined above,
wherein the reaction is carried out for 1 to 96 hours in aprotic solvent or in a heterogeneous mixture at 0-210 °C in the presence of base.

4. A method for preparation according to claim 3, **characterized in that** it is optionally conducted under phase transfer catalysis (PTC) conditions.

5. A method for preparation according to claim 3 or 4, **characterized in that** the aprotic solvent is selected from the group comprising: acetonitrile, dichloromethane, dichloroethane, dimethylformamide, dimethylacetamide, benzene, toluene, *ortho*-xylene, *meta*-xylene, *para-*xylene, tetrahydrofuran, 2-methyltetrahydrofuran, 1,2-dimethoxyethane, 1,4-dioxane, *tert-*butyl methyl ether, N-methylpyrrolidone, dimethylsulfoxide, or the mixture thereof.

6. A method for preparation according to claim 3 or 4, **characterized in that** the heterogeneous mixture comprises: dichloromethane, dichloroethane, benzene, toluene, *ortho*-xylene, *meta-*xylene, *para*-xylene, *tert*-butyl methyl ether, 2-methyltetrahydrofuran, or the mixture thereof as the organic phase, and the aqueous solution of alkali metal hydroxides.

7. A method for preparation according to any of claims 3-6, **characterized in that** the base is selected from the group comprising alkali metal carbonates (M₂CO₃), alkali metal hydroxides (MOH), alkali metal hydrides (MH), and alkali metal phosphates (M₃PO₄), where M = Li, Na, K, Cs, and alkali metal alkoxides (ROM), where M = Na, K, and R is a C₁-C₄ alkyl group, specifically selected from methyl, ethyl, n-propyl and isopropyl.

8. A method for preparation according to claim 7, **characterized in that** the alkali metal carbonates (M₂CO₃), alkali metal hydroxides (MOH) and alkali metal phosphates (M₃PO₄) are in the solid form or as aqueous solution.

9. A method for the selective extraction of lithium from a solid phase into a liquid organic phase using the molecular receptor specified in claim 1 or 2, comprising the steps of:
(a) Contacting the solid material containing lithium salts with a solution of the molecular receptor in the range of 0.001-2 mol/L in a liquid organic phase;
(b) Allowing the system to equilibrate from 1 second to 30 days to enable lithium transfer from the solid phase into the liquid organic phase;
(c) Separating the liquid organic phase from the solid residue by decantation, filtration, or centrifugation;
(d) Optionally filtering the organic phase to remove any suspended particulates,
wherein the whole process is conducted at a temperature ranging from 0°C to 100°C, preferably between 20°C and 40°C.

10. A method for the selective extraction according to claim 9, **characterized in that** the solid material is selected from individual lithium salts, salt mixtures, or spent lithium-ion battery materials.

11. A method for the selective extraction according to claim 9 or 10, **characterized in that** the extraction process is conducted under controlled mixing conditions, including continuous agitation, intermittent shaking, or passive equilibration over time.

12. A method for the selective extraction according to any of claims 9 -11, **characterized in that** the process is performed in a batch-wise or continuous mode.

13. A method for the selective extraction according to any of claims 9-12, **characterized in that** the liquid organic phase is selected from the group comprising chlorinated hydrocarbons, branched chlorinated hydrocarbons (C₄-C₁₈), hydrocarbons (C₅-C₁₇), branched hydrocarbons (C₅-C₁₈), toluene, benzene, nitrobenzene, ortho-xylene, meta-xylene, para-xylene, or the mixture thereof.

14. A method for the selective extraction according to any of claims 9-13, **characterized in that** a solid phase is selected from the group comprising lithium salts.

15. A use of the molecular receptor specified in claim 1 or 2 for the recovery of lithium from primary and secondary sources, including the recycling of lithium-ion batteries.
